# EUROPÄISCHE PATENTSCHRIFT

(11) **EP 2 153 222 B1**
(45) Veröffentlichungstag und Bekanntmachung des Hinweises auf die Patenterteilung: **30.11.2011**
(21) Anmeldenummer: 08736010.3
(22) Anmeldetag: 09.04.2008
(51) Int. Cl.: G01N 33/34, G01N 23/06

(54) **VERFAHREN UND VORRICHTUNG ZUR MESSUNG DER ZUSAMMENSETZUNG VON FASERSTOFFSUSPENSIONEN**
METHOD AND DEVICE FOR MEASURING THE COMPOSITION OF FIBROUS SUSPENSIONS
PROCÉDÉ ET DISPOSITIF DE MESURE DE LA COMPOSITION DE SUSPENSIONS DE MATIÈRE FIBREUSE

(30) Priorität: 01.06.2007 DE 102007025709
(43) Veröffentlichungstag der Anmeldung: 17.02.2010
(73) Patentinhaber: Voith Patent GmbH, 89522 Heidenheim (DE)
(72) Erfinder: TRUE, Marcus, 88212 Ravensburg (DE)
(86) Internationale Anmeldenummer: PCT/EP2008/054285
(87) Internationale Veröffentlichungsnummer: WO 2008/145449

(56) Entgegenhaltungen:
- DE-A1- 10 313 116
- GB-A- 2 088 050
- GB-A- 2 227 311
- US-A- 4 239 590
- US-A- 4 916 719

## Beschreibung

Die vorliegende Erfindung betrifft ein Verfahren und eine Vorrichtung zur Messung der Zusammensetzung von Faserstoffsuspensionen, insbesondere in einer Papiermaschine.

Die Messung der Zusammensetzung von Papiersuspensionen wird durch unterschiedliche Aschegehalte erschwert. Die Messung des Aschegehaltes ist ebenfalls schwierig, eine zuverlässige Methode bislang nicht bekannt.

Gegenwärtig wird die Gesamtzusammensetzung von Faserstoffsuspensionen zumeist mittels rotierender Sensoren und optischen Methoden, die im Infraroten arbeiten, durchgeführt. Rotierende Sensoren sind aber nur bei festem Aschegehalt einsetzbar, da hiermit der Fasergehalt gemessen wird. Ein weiteres Problem in Deinkinganlagen ist die starke Abhängigkeit von der Faserlängenverteilung: selbst wenn nur eine Papiersorte hergestellt wird, variiert die Zusammensetzung des Ausgangsmaterials. Optische Sensoren sind stark abhängig vom Gehalt an Druckerschwärze, was insbesondere in Deinkinganlagen problematisch ist.

Ein weiteres. Problem ist die wesentliche Abhängigkeit vom Gasgehalt, die nicht kompensiert werden kann. Der Einfluss von Gasblasen auf die Lichtstreuung ist stark von der Größenverteilung abhängig. Es besteht keine lineare Beziehung zu dem tatsächlichen Gasgehalt in Prozent. Bei hohen Gasanteilen liefern optische Methoden daher keine brauchbaren Ergebnisse.

In der DE10313116 wird die Gammastrahlungsabsorption einer Faserstoffsuspension gemessen, um die mittlere Dichte zu bestimmen.

Der Erfindung liegt die Aufgabe zugrunde, eine zuverlässige Messung der Zusammensetzung von Faserstoffsuspensionen zu ermöglichen, mit der auch der Aschegehalt bestimmt werden kann und die auch im laufenden Verfahren einsetzbar ist.

Der Aschegehalt der Suspension wird erfindungsgemäß mittels Gammastrahlung von Curium 244 bestimmt, während der Gesamtfasergehalt bevorzugt mittels Gammastrahlung von Cäsium 137 bestimmt wird. Die Erfindung basiert nämlich auf der Erkenntnis, dass Curium 244 als Strahlungsquelle eine Messung der mittleren Aschendichte in einer Faserstoffsuspension ermöglicht, während der Fasergehalt zu einer Absorption im Bereich von Wasser führt. Hintergrund ist der Absorptionskoeffizient für Gammastrahlung, der für Elemente höherer Ordnung ansteigt. Daher haben Wasser und Fasern eine ähnliche mittlere Absorption und führen zur gleichen Dämpfung der Gammastrahlen. Weiteren Vorteil zieht die Erfindung daraus, dass Druckerschwärze hauptsächlich aus Kohlenstoff besteht und der Gehalt an Druckerschwärze daher zu einer Absorption ähnlich von Wasser und Fasern führt. Als Folge ist der Aschegehalt der einzig verbleibende Anteil und kann daher mit guter Genauigkeit gemessen werden.

Ein weiterer Vorteil der Erfindung besteht darin, dass Sensoren für Gammastrahlen von Haus aus unempfindlich für Farbe, Druckerschwärze oder ähnliches sind. Auch besteht keine Abhängigkeit vom Durchfluss. Vom Gasgehalt besteht andererseits eine definierte Abhängigkeit, so dass die Möglichkeit einer Korrektur gegeben ist. Beispielsweise erniedrigt sich die Dichte bei einem Volumenanteil an Gas von 10 % ebenfalls um 10 %.

Dementsprechend werden die erhaltenen Messwerte bevorzugt in Abhängigkeit vom Gasgehalt der Suspension korrigiert. Ist der Gasgehalt nicht bekannt, so wird dieser nach einer Ausgestaltung der Erfindung gemessen, insbesondere während des Betriebs der Papiermaschine.

Eine weitere Korrektur kann in Abhängigkeit vom Druck erfolgen, insbesondere wenn sich dieser stark ändert.

Eine bevorzugte weitere Korrekturmöglichkeit besteht darin, die Messwerte in Abhängigkeit von der Temperatur zu korrigieren. Die Messgenauigkeit kann dadurch weiter erhöht werden.

Eine noch weitere Erhöhung der Messgenauigkeit kann dadurch erreicht werden, dass auch die Änderung der Strahlungsintensität kompensiert wird, insbesondere auf elektronischem Wege.

Eine besonders bevorzugte Anwendung der Erfindung sieht vor, dass die Messung im Bereich der Flotation einer Papiermaschine durchgeführt wird. Trotz des dort herrschenden hohen und variierenden Gasanteils können hier aufgrund der Erfindung Messungen mit guter Genauigkeit durchgeführt werden, was bislang überhaupt nicht möglich war.

Besonders bevorzugt ist es dabei, wenn die Zusammensetzung der Suspension, inklusive Aschegehalt, sowohl im Durchfluss als auch in der Abführleitung gemessen wird. Damit können der Flotationsprozess optimiert und die Kosten gesenkt werden. Die Flotation, die dazu dient, unerwünschte Stoffe wie Druckerschwärze und Klebstoffe zu entfernen, führt nämlich auch dazu, dass Ascheanteile mit abgeführt werden. Das Ersetzen dieser Anteile ist kostenintensiv. Auch erhöht der Ascheanteil im Reject die Entsorgungskosten.

Gemäß einer weiteren Ausgestaltung der Erfindung wird daher der Aschegehalt aufgrund der erhaltenen Messwerte geregelt. Damit kann die genannte Kostenreduzierung erreicht werden.

Die Messung des Gasgehaltes der Suspension erfolgt bevorzugt durch Schallwellen. Derartige Messverfahren sind bekannt und haben sich bewährt.

Eine erfindungsgemäße Vorrichtung umfasst die in den Vorrichtungsansprüchen enthaltenen Merkmale.

Ausführungsbeispiele der Erfindung sind in den Zeichnungen dargestellt und werden nachfolgend beschrieben. Es zeigen, jeweils in schematischer Darstellung
- Fig. 1: eine Anordnung zur Messung der Zusammensetzung von Faserstoffsuspensionen im Bereich der Flotation einer Deinkinganlage; und
- Fig. 2: einen Ausschnitt aus einer Anlage mit erfindungsgemäßer Messeinrichtung.

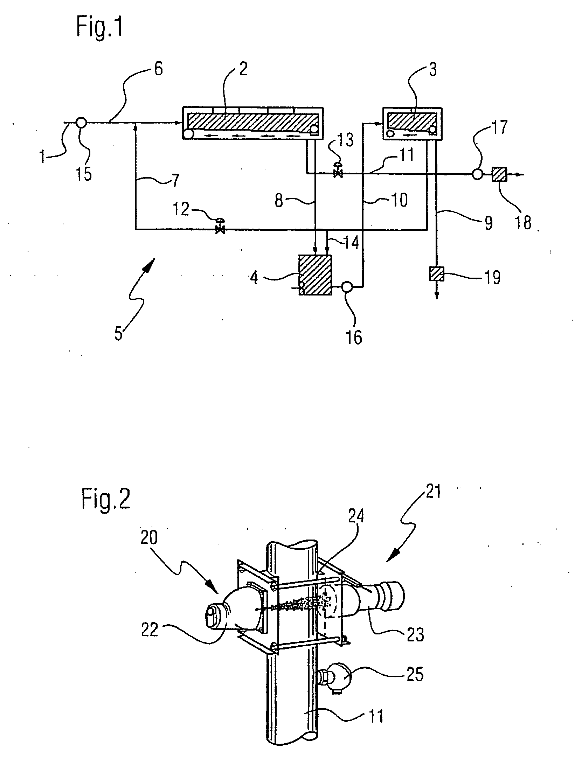

Die in Fig. 1 dargestellte Anlage umfasst einen Zulauf 1 für die Suspension, eine Primärstufe 2 der Flotation, eine Sekundärstufe 3 der Flotation, einen Sammler 4 und eine Verrohrung 5 zur Verbindung der genannten Bestandteile, umfassend eine Zulaufleitung 6, eine Rücklaufleitung 7 von der Sekundärstufe 3, eine Ablaufleitung 8 von der Primärstufe 2, eine Ablaufleitung 9 von der Sekundärstufe 3, eine Verbindungsleitung 10 zwischen Sammler 4 und Sekundärstufe 3 sowie eine Durchflussleitung 11.

In der Rücklaufleitung 7 ist ein Absperrventil 12, und in der Durchflussleitung 11 ein Absperrventil 13 angeordnet. Des Weiteren besteht noch eine Ablaufverbindung 14 zwischen der Rücklaufleitung 7 und dem Sammler 4. Außerdem ist in der Zulaufleitung 6 eine Pumpe 15, in der Verbindungsleitung 10 eine Pumpe 16 und in der Durchflussleitung 11 eine Pumpe 17 vorgesehen.

Erfindungsgemäß ist eine erste Messvorrichtung 18 in der Durchflussleitung 11 und eine zweite Messvorrichtung 19 in der Abflussleitung 9 vorgesehen. Die Messvorrichtungen 18 und 19 arbeiten jeweils mit Gammastrahlung in Absorption. Sie umfassen mindestens eine, bevorzugt zwei Gammastrahlungsquellen, insbesondere Curium 244 und Cäsium 137, sowie entsprechende Sensoren. Die beiden Strahlungsquellen und die beiden Sensoren können jeweils in einem gemeinsamen Gehäuse angeordnet sein.

Fig. 2 zeigt eine beispielhafte Anordnung einer erfindungsgemäßen Messvorrichtung an einem Rohr, beispielsweise dem Durchflussrohr 11 von Fig. 1. Die Messvorrichtung umfasst eine Gammastrahlungsquelle 20, beispielsweise eine Cäsium 137-Strahlungsquelle sowie einen entsprechenden Sensor 21. Strahlungsquelle 20 und Sensor 21 sind auf voneinander abgewandten Seiten der Rohrleitung 11 an dieser angebracht. Hierfür sind die Strahlungsquelle 20 und der Sensor 21 jeweils in einem Gehäuse 22 bzw. 23 untergebracht, die über Spannmittel 24 so miteinander verbunden sind, dass sie auf der Rohrleitung 11. festgelegt sind. Zusätzlich kann in dem Gehäuse 22 noch eine Curium-244-Strahlungsquelle und in dem Gehäuse 23 ein entsprechender Sensor angeordnet sein.

Mit der dargestellten Anordnung kann die Zusammensetzung der durch die Rohrleitung 11 fließenden Faserstoffsuspension im Betrieb gemessen werden, insbesondere ihr Aschengehalt und/oder Gesamtfaserstoffgehalt. Die Messwerte können insbesondere für eine Regelung des Aschegehaltes in der Flotation einer Deinkinganlage eingesetzt werden. Durch die Verwendung einer Gammastrahlungsquelle ergeben sich gute Messergebnisse. Erforderlichenfalls können die Messwerte noch bezüglich des Gasgehalts, des Drucks und/oder der Temperatur der Faserstoffsuspension korrigiert werden. In diesem Fall sind weitere Messeinrichtungen für die genannten Parameter vorgesehen, insbesondere eine Schallmesseinrichtung 25 zur Messung des Gasgehalts. Außerdem kann die Abnahme der Strahlungsintensität der Strahlungsquelle 20 mit der Zeit kompensiert werden, insbesondere auf elektronischem Weg.

### Bezugszeichenliste

- 1: Zulauf
- 2: Primärstufe
- 3: Sekundärstufe
- 4: Sammler
- 5: Verrohrung
- 6: Zulaufleitung
- 7: Rücklaufleitung
- 8: Ablaufleitung
- 9: Ablaufleitung
- 10: Verbindungsleitung
- 11: Durchflussleitung
- 12: Absperrventil
- 13: Absperrventil
- 14: Ablaufleitung
- 15: Pumpe
- 16: Pumpe
- 17: Pumpe
- 18: Messeinrichtung
- 19: Messeinrichtung
- 20: Strahlungsquelle
- 21: Sensor
- 22: Gehäuse
- 23: Gehäuse
- 24: Befestigungsmittel
- 25: Schallmesseinrichtung

## Patentansprüche

1. Verfahren zur Messung der Zusammensetzung von Faserstoffsuspensionen, insbesondere in einer Papiermaschine, bei dem zur Messung der Zusammensetzung der Faserstoffsuspension Gammastrahlung verwendet wird
**dadurch gekennzeichnet,**
**dass** der Aschegehalt der Suspension gemessen und zur Messung des Aschegehalts Gammastrahlung von Curium 244 verwendet wird.

2. Verfahren nach Anspruch 1,
**dadurch gekennzeichnet,**
**dass** die Absorption der Gammastrahlung durch die Suspension gemessen wird.

3. Verfahren nach einem der vorhergehenden Ansprüche,
**dadurch gekennzeichnet,**
**dass** der Fasergehalt gemessen wird

4. Verfahren nach Anspruch 3,
**dadurch gekennzeichnet,**
**dass** zur Messung des Fasergehalts, Strahlung von Cäsium 137 verwendet wird

5. Verfahren nach einem der vorhergehenden Ansprüche,
**dadurch gekennzeichnet,**
**dass** die Messung während des Betriebs der Papiermaschine an einem von der Suspension durchströmten Rohr (11) durchgeführt wird.

6. Verfahren nach einem der vorhergehenden Ansprüche,
**dadurch gekennzeichnet,**
**dass** die Messwerte in Abhängigkeit vom Gasgehalt der Suspension korrigiert werden, wobei der Gasgehalt gemessen wird, insbesondere während des Betriebs der Papiermaschine.

7. Verfahren nach Anspruch 6,
**dadurch gekennzeichnet,**
**dass** die Messung des Gasgehalts der Suspension durch Schallmessung erfolgt und /oder die Messwerte in Abhängigkeit vom Druck in der Suspension korrigiert werden.

8. Verfahren nach einem der vorhergehenden Ansprüche,
**dadurch gekennzeichnet,**
**dass** die Messung im Bereich einer Flotation durchgeführt wird, insbesondere in der Durchflussleitung (11) und /oder der Ablaufleitung (9) der Flotation.

9. Verfahren nach einem der vorhergehenden Ansprüche,
**dadurch gekennzeichnet,**
**dass** die erhaltenen Messwerte zur Steuerung oder Regelung des Aschegehaltes verwendet werden.

10. Vorrichtung zur Messung der Zusammensetzung von Faserstoffsuspensionen, insbesondere in einer Papiermaschine, die eine Gammastrahlungsquelle (20) und einen Gammastrahlungssensor (21) umfasst
**dadurch gekennzeichnet,**
**dass** die Vorrichtung eine Curium 244 Strahlungsquelle aufweist.

11. Vorrichtung nach Anspruch 10
**dadurch gekennzeichnet,**
**dass** die Vorrichtung Zusätzlich eine Cäsium 137 Strahlungsquelle aufweist.

12. Vorrichtung nach einem der Ansprüche 10 oder 11,
**dadurch gekennzeichnet,**
**dass** die Vorrichtung zur Messung der Fasergehaltes ausgebildet ist.

13. Vorrichtung nach einem der Ansprüche 10 bis 12,
**dadurch gekennzeichnet,**
**dass** die Vorrichtung zur Messung während des Betriebs der Papiermaschine ausgebildet ist und die Vorrichtung an einem von der Suspension durchströmten Rohr (11) angeordnet ist.

14. Vorrichtung nach einem der Ansprüche 10 bis 13,
**dadurch gekennzeichnet,**
**dass** Mittel zur Korrektur der Messwerte in Abhängigkeit vom Gasgehalt und /oder zur Korrektur der Messwerte in Abhängigkeit vom Druck und/oder in Abhängigkeit von der Temperatur in der Suspension und/oder in Abhängigkeit von der Strahlungsintensität der Strahlungsquelle vorgesehen sind.

15. Vorrichtung nach Anspruch 14
**dadurch gekennzeichnet,**
**dass** eine Schallmesseinrichtung (25) zur Messung des Gasgehalts vorgesehen ist.

16. Vorrichtung nach einem der Ansprüche 10 bis 15
**dadurch gekennzeichnet**,
Mittel zur Steuerung und/oder Regelung des Aschegehalts aufgrund der erhaltenen Messwerte vorgesehen sind.

17. Papiermaschine mit einer Vorrichtung nach einem der Ansprüche 10 bis 16.

## Claims

1. Method for measuring the composition of fibrous suspensions, in particular in a papermaking machine, in which gamma radiation is used to measure the composition of the fibrous suspension,
**characterized in that**
the ash content of the suspension is measured and gamma radiation from curium 244 is used to measure the ash content.

2. Method according to Claim 1,
**characterized in that**
the absorption of the gamma radiation by the suspension is measured.

3. Method according to one of the preceding claims,
**characterized in that**
the fibre content is measured.

4. Method according to Claim 3,
**characterized in that**
in order to measure the fibre content, radiation from caesium 137 is used.

5. Method according to one of the preceding claims,
**characterized in that**
the measurement is carried out during the operation of the papermaking machine on a pipe (11) through which the suspension flows.

6. Method according to one of the preceding claims,
**characterized in that**
the measured values are corrected as a function of the gas content of the suspension, the gas content being measured, in particular during the operation of the papermaking machine.

7. Method according to Claim 6,
**characterized in that**
the measurement of the gas content of the suspension is carried out by means of sound measurement and/or the measured values are corrected as a function of the pressure in the suspension.

8. Method according to one of the preceding claims,
**characterized in that**
the measurement is carried out in the area of a flotation system, in particular in the flow line (11) and/or the drain line (9) of the flotation system.

9. Method according to one of the preceding claims,
**characterized in that**
the measured values obtained are used for the open-loop or closed-loop control of the ash content.

10. Device for measuring the composition of fibrous suspensions, in particular in a papermaking machine, which comprises a gamma radiation source (20) and a gamma radiation sensor (21),
**characterized in that**
the device has a curium 244 radiation source.

11. Device according to Claim 10,
**characterized in that**
the device additionally has a caesium 137 radiation source.

12. Device according to either of Claims 10 and 11,
**characterized in that**
the device is designed to measure the fibre content.

13. Device according to one of Claims 10 to 12,
**characterized in that**
the device is designed to measure during the operation of the papermaking machine, and the device is arranged on a pipe (11) through which the suspension flows.

14. Device according to one of Claims 10 to 13,
**characterized in that**
means are provided to correct the measured values as a function of the gas content and/or to correct the measured values as a function of pressure and/or as a function of the temperature in the suspension and/or as a function of the radiation intensity of the radiation source.

15. Device according to Claim 14,
**characterized in that**
a sonic device (25) is provided to measure the gas content.

16. Device according to one of Claims 10 to 15,
**characterized in that**
means are provided for the open-loop and/or closed-loop control of the ash content on the basis of the measured values obtained.

17. Papermaking machine having a device according to one of Claims 10 to 16.

## Revendications

1. Procédé de mesure de la composition de suspensions de matière fibreuse, en particulier dans une machine à papier, dans lequel on utilise un rayonnement gamma pour la mesure de la composition de la suspension de matière fibreuse, **caractérisé en ce que** l'on mesure la teneur en cendres de la suspension et on utilise le rayonnement gamma du curium 244 pour la mesure de la teneur en cendres.

2. Procédé selon la revendication 1, **caractérisé en ce que** l'on mesure l'absorption du rayonnement gamma par la suspension.

3. Procédé selon l'une quelconque des revendications précédentes, **caractérisé en ce que** l'on mesure la teneur en fibres.

4. Procédé selon la revendication 3, **caractérisé en ce que** l'on utilise le rayonnement du césium 137 pour la mesure de la teneur en fibres.

5. Procédé selon l'une quelconque des revendications précédentes, **caractérisé en ce que** l'on effectue la mesure sur un tube (11) parcouru par la suspension pendant le fonctionnement de la machine à papier.

6. Procédé selon l'une quelconque des revendications précédentes, **caractérisé en ce que** l'on corrige les valeurs de mesure en fonction de la teneur en gaz de la suspension, dans lequel on mesure la teneur en gaz, en particulier pendant le fonctionnement de la machine à papier.

7. Procédé selon la revendication 6, **caractérisé en ce que** l'on effectue la mesure de la teneur en gaz de la suspension par une mesure sonore et/ou on corrige les valeurs de mesure en fonction de la pression dans la suspension.

8. Procédé selon l'une quelconque des revendications précédentes, **caractérisé en ce que** l'on effectue la mesure dans la région d'une flottation, en particulier dans la conduite de circulation (11) et/ou dans la conduite d'évacuation (9) de la flottation.

9. Procédé selon l'une quelconque des revendications précédentes, **caractérisé en ce que** l'on utilise les valeurs de mesure obtenues pour la commande ou la régulation de la teneur en cendres.

10. Dispositif de mesure de la composition de suspensions de matière fibreuse, en particulier dans une machine à papier, qui comprend une source de rayonnement gamma (20) et un détecteur de rayonnement gamma (21), **caractérisé en ce que** le dispositif présente une source de rayonnement de curium 244.

11. Dispositif selon la revendication 10, **caractérisé en ce que** le dispositif présente en outre une source de rayonnement de césium 137.

12. Dispositif selon l'une quelconque des revendications 10 ou 11, **caractérisé en ce que** le dispositif est conçu pour la mesure de la teneur en fibres.

13. Dispositif selon l'une quelconque des revendications 10 à 12, **caractérisé en ce que** le dispositif est conçu pour la mesure pendant le fonctionnement de la machine à papier et le dispositif est disposé sur un tube (11) parcouru par la suspension.

14. Dispositif selon l'une quelconque des revendications 10 à 13, **caractérisé en ce qu'**il est prévu des moyens pour la correction des valeurs de mesure en fonction de la teneur en gaz et/ou pour la correction des valeurs de mesure en fonction de la pression et/ou en fonction de la température dans la suspension et/ou en fonction de l'intensité du rayonnement de la source de rayonnement.

15. Dispositif selon la revendication 14, **caractérisé en ce qu'**il est prévu un dispositif de mesure sonore (25) pour la mesure de la teneur en gaz.

16. Dispositif selon l'une quelconque des revendications 10 à 15, **caractérisé en ce qu'**il est prévu des moyens pour la commande et/ou la régulation de la teneur en cendres sur la base des valeurs de mesure obtenues.

17. Machine à papier équipée d'un dispositif selon l'une quelconque des revendications 10 à 16.
